# EUROPÄISCHE PATENTSCHRIFT

(11) **EP 1 773 483 B1**
(45) Veröffentlichungstag und Bekanntmachung des Hinweises auf die Patenterteilung: **06.02.2008**
(21) Anmeldenummer: 05753654.2
(22) Anmeldetag: 28.05.2005
(51) Int. Cl.: B01J 8/08, C01C 1/04, C07C 29/151

(54) **VERFAHREN ZUR DURCHFÜHRUNG HETEROGEN KATALYTISCHER EXOTHERMER GASPHASENREAKTIONEN**
METHOD FOR CARRYING OUT HETEROGENEOUS CATALYTIC EXOTHERMIC GAS PHASE REACTIONS
PROCEDE POUR EFFECTUER DES REACTION EN PHASE GAZEUSE EXOTHERMIQUES CATALYTIQUES HETEROGENES

(30) Priorität: 28.05.2004 DE 102004028200
(43) Veröffentlichungstag der Anmeldung: 18.04.2007
(73) Patentinhaber: Ammonia Casale S.A., 6900 Lugano-Besso (CH)
(72) Erfinder: HIPP, Anton, Josef, 97082 Würzburg (DE)
(74) Vertreter: Zardi, Marco
(86) Internationale Anmeldenummer: PCT/EP2005/005775
(87) Internationale Veröffentlichungsnummer: WO 2005/115607

(56) Entgegenhaltungen:
- EP-A- 0 268 469
- EP-A- 0 682 002
- EP-A- 0 873 972
- US-A- 5 216 034

## Beschreibung

Die vorliegende Erfindung betrifft ein Verfahren zur Durchführung heterogen katalytischer exothermer Gasphasenreaktionen, unter adiabatischen und nichtadiabatischen Bedingungen, im Hinblick auf größere Umwandlungsausbeute bei geringerem Energieverbrauch. Beispiele für solche exothermen heterogenen katalytischen Gasphasenreaktionen sind etwa die Herstellung von Methanol aus Synthesegas, die Herstellung von Ammoniak aus Wasserstoff und Stickstoff enthaltenden Gemischen oder die Herstellung von höheren Alkoholen aus Synthesegas.

Die industrielle Herstellung dieser Produkte basiert auf stark exothermen Gleichgewichtsreaktionen, die für die Herstellung dieser Produkte weniger günstig sind, wobei vernünftige Reaktionsausbeuten nur durch Verwendung von selektiven Katalysatoren möglich sind. Die Herstellung dieser Produkte wird aus der Sicht des chemischen Gleichgewichts durch relativ niedrige Temperaturen begünstigt. Die Reaktionsgeschwindigkeit ist abhängig von der Aktivität des Katalysators, von der Temperatur, und von der Zusammensetzung des Synthesegases.

Unter bestimmten Reaktionsbedingungen kann die Temperatur im Katalysatorbett ein akzeptables Niveau überschreiten und so eine Beschädigung des Katalysators verursachen. Das Kühlen des Katalysatorbettes kann entweder durch Außenkühlen des Konverters durchgeführt werden, was im wesentlichen einem isothermen Betrieb entspricht, oder durch Zwischenkühlen zwischen aufeinander folgenden, adiabatischen Katalysatorbetten in einem oder mehreren Konvertern. Die adiabatischen Verfahren haben kleinere Umsätze pro Durchgang als die isothermen Verfahren, so dass sie größere Katalysatorvolumen und größere Kreislaufverhältnisse benötigen.

Der technische Hintergrund der vorliegenden Erfindung wird zunächst anhand der Methanolsynthese erläutet. Bei der Methanolsynthese sind insbesondere folgende Reaktionen von Bedeutung:

| | |
|---|---|
| CO + 2H₂ | CH₃OH - 90,5 KJ/mol (1) |
| CO₂ + 3 H₂ | CH₃OH + H₂O - 49,4 KJ/mol (2) |
| CO₂ + H₂ | CO + H₂O + 41,1 KJ/mol (3) |

Gegenwärtig werden bei der industriellen Herstellung von Methanol nur Katalysatoren auf der Basis von CuO/ZnO/Al₂O₃ verwendet. Die Synthese wird bei Drücken im Bereich von 20 bis 120 bar und Temperaturen im Bereich von 200 bis 350° C durchgeführt.

Das Synthesegas, bestehend aus einem Gemisch von Wasserstoff mit Kohlenmonoxid und Kohlendioxid, wird durch Dampfreformieren oder autothermisches Reformieren von Erdgas erhalten. Es wird mit Umlaufgas gemischt, vorerhitzt und bis zum Synthesedruck verdichtet, wobei das Gas in einem nachfolgenden Reaktor reagiert. Die herkömmlichen Methanolreaktoren bestehen aus einem Druckgefäß, das mit einem oder mehreren Synthesekatalysatorbetten, Kühlgaseinheiten und/oder Wärmeaustauschern ausgestattet ist, um das Synthesegas vorzuwärmen und die Reaktionswärme zu kontrollieren.

Aus dem ausströmenden Reaktorgas wird rohes Methanol kondensiert, und das unreagierte Gas wird zum Einlass des Synthesereaktors zurückgeführt. Eine kleine Menge wird aus dem Kreislauf entfernt, um den Gehalt an Inertgasen zu begrenzen. Zum Stand der Technik gehören die sogenannten "Quench-Gas"-Reaktoren, in denen der Synthesekatalysator mit kalter Synthesegaseinführung kontrolliert wird. Beispiele von solchen Reaktoren wurden in den Patentschriften GB 1 105 614, EP 0 359 952, und US 4,859,425 beschrieben.

Diese Reaktoren und die entsprechenden Verfahren benötigen große Kreislaufverhältnisse, um große Umsätze zu erreichen. In der US 4,778,662 ist ein Reaktor, in dem die entstehende Wärme dem eintretenden Synthesegas übertragen wird, beschrieben. In den Patentschriften US 5,252,609 und US 5,631,302 ist ein Verfahren, in dem das Frischgas einer Vorsynthese unterworfen ist, um die Umlaufkosten zu reduzieren, beschrieben. In DE 196 05 572 A1 werden im Kreislauf zwei in Serie geschaltete Synthesereaktoren benutzt, um die Umlaufkosten zu reduzieren. Die hohen Kohlenoxidumsätze werden mit Frischgasen, deren CO₂-zu-CO-Verhältnisse kleiner als 0,5 sind, erreicht. Auch in der WO 99/59945 wird ein Verfahren beschrieben, in dem man die Kreislaufreduzierung durch 2 oder 3 in Serie geschaltete Reaktoren und Optimierung der Frischgas-Verteilung erreicht. Auch in diesen Fall wird Frischgas mit CO₂-zu-CO-Verhältnissen unter 0,5 verwendet. Diese Synthesegase können nur mit Autothermen oder Kombi-Reformieranlagen, die teuere Sauerstoffanlagen benötigen, hergestellt werden.

Der technische Hintergrund der Erfindung ist bei Anlagen zur Herstellung von Ammoniak ähnlich. Die EP 1 339 641 B1 beschreibt beispielsweise ein Verfahren, wo Ammoniak nach der Gleichung

| | |
|---|---|
| N₂ + 3 H₂ | 2 NH₃- 92,3kJ/mol (4) |

unter verschiedenen Drucken aus Synthesegas erzeugt wird. Hierzu wird das Synthesegas durch ein oder mehrere in Serie geschaltete Synthesesysteme, welche nicht als Kreislaufsysteme ausgeführt sind, geleitet, und anschließend in ein Kreislaufsystem eingeschleust. Hinter jedem dieser Synthesesysteme findet eine Druckanhebung statt und in jedem dieser Systeme wird erzeugtes Ammoniak gewonnen und ausgeschleust.

Als Synthesereaktoren werden üblicherweise Reaktoren nach der Art der DE 37 08 781 verwendet, die sowohl Katalysator als auch Wärmetauscher enthalten. Es ist bekannt, mehrere solcher Reaktoren in Serie zu schalten, wobei stets darauf geachtet werden muss, dass die Reaktoreintrittstemperatur stets einen minimale Temperatur aufweisen muss, etwa 300 bis 400 °C für die gebräuchlichsten Katalysatormaterialien, die Temperatur am Reaktoraustritt aber eine den Katalysator schädigende Maximaltemperatur nicht übersteigen darf. Ferner bewirkt jeder Temperaturanstieg stets eine Verschlechterung des aufgrund des chemischen Gleichgewichts erreichbaren Reaktorumsatzes. Man ist daher bestrebt, die Reaktortemperatur möglichst nah an der Minimaltemperatur zu führen.

Zur Einstellung dieser Reaktortemperaturen existieren eine Reihe von Verfahren. In der EP 272 449 wird ein Verfahren beschrieben, bei dem innerhalb eines Kreislaufsystems mindestens 2 Reaktoren in Reihe geschaltet werden, und vor dem letzten Reaktor zur Regelung wenigstens eine Teilmenge des das vorletzte Katalysatorbett verlassenden Kreislaufgases einem Dampferzeuger als Wärmetauscher und/oder einer diesen Wärmetauscher umgehenden Bypass-Leitung zugeführt wird, wobei eine Teilmenge kalten Kreislaufgases vor Aufheizung und Zuführung zum ersten Katalysatorbett der genannten Bypass-Leitung als Quentschmittel zuführbar ist.

Die für die Ammoniakherstellung genannten Verfahren weisen jedoch den Nachteil auf, dass die erzeugbare Ammoniakmenge durch das chemische Reaktionsgleichgewicht im letzten Reaktor beschränkt wird. Da diese Grenze oft schon nach 2 oder 3 Reaktoren erreicht ist, lassen sich nach dem bekannten Stand der Technik keine weiteren Reaktoren anschließen, wenn der Wunsch nach Kapazitätserweiterung besteht. Auch ist die Temperaturregelung bei wechselnden Betriebsbedingungen aufwändig und kompliziert.

Der vorliegenden Erfindung liegt die Aufgabe zugrunde, ein verbessertes Verfahren zur Durchführung heterogen katalytischer exothermer Gasphasenreaktionen bei erhöhter Temperatur und erhöhtem Druck zur Verfügung zu stellen, welches gleichermaßen auf Anlagen zur Herstellung von Methanol und Ammoniak sowie ähnlicher Synthesen von höheren Alkoholen aus Synthesegas angewendet werden kann und auch zur Nachrüstung bestehender Anlagen geeignet ist.

Die Erfindung löst die Aufgabe gemäß dem Hauptanspruch, indem man das Synthesegas, bestehend aus einem Gemisch von Frischgas und/oder Umlaufgas durch mindestens zwei in einen Synthesesystem in Serie geschaltete Synthesestufen leitet, wobei
- die Produktgase aus den Synthesestufen, mit Ausnahme der letzten Stufe, in mindestens zwei Teilströme getrennt werden,
- wobei ein Teilstrom soweit gekühlt wird, bis Produkt auskondensiert und das produkthaltige Kondensat von den gasförmigen Bestandteilen getrennt wird,
- und anschließend die gasförmigen Bestandteile mit dem warmen Teil des Produktgases zusammengeführt werden, um die Eintrittstemperatur der nächsten Synthesestufe zu erreichen

In Ausgestaltungen des Verfahrens wird der Teilstrom, aus dem Produkt auskondensiert und entfernt wurde, vor seiner Wiederzumischung zum warmem Teil des Produktgases erwärmt, wobei die Temperatur nach der Erwärmung unter der des warmem Teils des Produktgases liegt, sonst würde sich keine Verringerung der Mischtemperatur nach der Zusammenführung der Teilströme ergeben. Die Wärme, die zur Erwärmung des Teilstroms, der aus dem Produkt auskondensiert wurde, genutzt wird, kann diesem Teilstrom bei seiner Abkühlung wenigstens teilweise entzogen werden. Praktisch kann dies geschehen, indem ein Gas-Gas-Wärmetauscher vorgesehen wird.

Je nach den vor Ort gegebenen Rahmenbedingungen können jedoch auch andere Wärmeverschaltungen sinnvoll und möglich sein, insbesondere im Verbund mit den Verfahren, die zur Kondensation des Produktes und zur Herstellung des verwendeten Synthesegases eingesetzt werden.

In einer weiteren Ausgestaltung des Verfahrens wird aus dem abzukühlenden Teil des Produktgases der größte Teil der Wärme durch indirekten Wärmeaustausch mit Kesselspeisewasser und/oder voll entsalztem Wasser rückgewonnen.

In einer weiteren Ausgestaltung des Verfahrens besteht das in den ersten Synthesereaktor geleitete Synthesegas zu 16 bis 40 Mol.-% aus frischem Synthesegas. Eine weitere Zufuhr von Synthesegas zwischen den einzelnen Reaktoren des Reaktionssystems, welches in diesem Fall als Kreislaufsynthese ausgeführt sein muss, ist nicht vorgesehen.

In einer weiteren Ausgestaltung des Verfahrens wird der letzte Reaktor eines Synthesesystems in indirekten Wärmeaustausch mit Synthesegas gekühlt. Beispielsweise ist es bei der Synthese von Ammoniak üblich, statt dessen den ersten Reaktor zu kühlen, da dort die schärfsten Reaktionsbedingungen vorliegen - die Eingangs-Produktkonzentrationen sind so gering wie möglich, um bis zum Erreichen des Gleichgewichtsumsatz eine möglichst große Spanne vorzulegen - und damit der Temperaturanstieg aufgrund der Exothermie besonders steil verläuft. Dies ist bei der vorliegenden Erfindung nicht mehr erforderlich, da jeder der in Serie angeordneten Reaktoren mit annähernd den gleichen Eingangs-Produktkonzentrationen betrieben werden kann. Hierdurch wird es möglich, die Ausgangstemperatur des letzten Reaktors zu senken und dadurch das chemische Gleichgewicht der exothermen Reaktion zu einem günstigern Betriebspunkt hin zu verschieben, was die Ausbeute weiter erhöht.

Weitere Ausgestaltungen der Erfindung betreffen die Methanolsynthese im Speziellen. Hierbei wird aus einem Wasserstoff, Kohlenmonoxid und Kohlendioxid enthaltenden Synthesegas durch Umsetzung an körnigen, kupferhaltigen Katalysatoren bei Drücken im Bereich von 20 bis 120 bar und Temperaturen im Bereich von 200 bis 350° C Methanol erzeugt, wobei die Kondensation im Teilstrom zwischen den einzelnen Reaktoren derart eingestellt wird, dass sich zwischen der ersten und der letzten Synthesestufe 15 bis 50 % der gesamten Methanolproduktion in dem abgeschiedenen methanolhaltigen Kondensat befinden.

Hierbei werden erfindungsgemäß als jeweils erster Synthesereaktor entweder ein adiabater Mehrbettreaktor, ein Röhrenreaktor mit Synthesegaskühlung, oder ein isothermer Reaktor bevorzugt, im letzteren Fall sollte der Katalysator von Röhren, in denen unter erhöhtem Druck siedendes Wasser als Kühlmittel fließt, durchzogen sein.

Auf diese Weise ist es wirtschaftlich möglich, die Methanolsynthese aus Wasserstoff, Kohlenmonoxid und Kohlendioxid, in mindestens zwei Synthesestufen umsatzsteigernd und kostensenkend, auch bei CO₂-zu-CO-Verhältnissen bis zu 1:1 in Frischgas, durchzuführen. Auch wird auf diese Weise eine günstige Nachrüstbarkeit geschaffen, mit zusätzlichem, fremdem Synthesegas-Einsatz die Produktionskapazitäten in Kreislaufsystemen ohne zusätzlichen Umlaufkosten um bis zu 50 % zu steigern, indem man das Produktgemisch von der Auslasstemperatur einer Synthesestufe zur Einlasstemperatur der nächsten Synthesestufe abkühlt, und gleichzeitig aus dem Produktgemisch Rohmethanol entfernt. Dadurch dass man Methanol und Wasser aus dem Produktgemisch der Vorstufe entfernt, wird die Zusammensetzung des Synthesegases für der nächste Synthesestufe, günstiger zum Gleichgewicht eingestellt, und deswegen der CO und CO₂ Umsatz pro Durchgang gesteigert.

Unter Verwendung von zwei Synthesestufen ist ein erfindungsgemäßes Verfahren typischerweise dadurch geprägt, dass
1. man Frischgas und Umlaufgas in die erste Synthesestufe leitet,
2. man das erste Produktgemisch bis zur Eintrittstemperatur der Kühlröhren der zweiten Synthesestufe abkühlt, und so durch teilweise Kondensation 15% bis 50% der gesamten Methanolproduktion aus dem Synthesegas vor der zweiten Synthesestufe entfernt,
3. die zweite Synthesestufe, bzw. der zweite Synthesereaktor, mit Synthesegas in indirekten Wärmeaustausch gekühlt wird,
4. man das zweite Produktgemisch, aus der zweiten Synthesestufe, abkühlt und die Rohmethanoldämpfe kondensiert ,
5. man das vom methanolhaltigen Kondensat zurückführende Synthesegas abtrennt, und mit Frischgas aus einer externen Quelle mischt, mit dem zweiten Produktgemisch und teilweise auch mit dem ersten Produktgemisch bis zur Eintrittstemperatur in der ersten Synthesestufe vorwärmt,
6. die erste Synthesestufe bzw. der erste Synthesereaktor entweder als,
   a. ein adiabater Mehrbettreaktor,
   b. ein Reaktor mit indirekter Synthesegaskühlung,
   c. ein Reaktor in dem der Katalysator von Röhren durchzogen ist, in denen unter erhöhtem Druck siedendes Wasser als Kühlmittel fließt,
   ausgeführt sein kann.

Dies ermöglicht, auch für die bekannten Syntheseverfahren, mit einer Synthesestufe, durch Nachschaltung eines zweiten mit Synthesegas indirekt gekühlten Reaktors eine kostengünstige Nachrüstbarkeit zu schaffen, die Produktion zu steigern und die Kosten zu senken. Beim zusätzlichen Einsatz der Erfindung in den bekannten Verfahren mit adiabaten in Serie geschalteten Schachtreaktoren wird 10% bis 40% der gesamten Methanolproduktion zwischen der ersten und der letzten Synthesestufe aus dem Produktgas durch teilweise Kondensation entzogen. Dies geschieht wegen des kleinen Temperaturabstands zwischen Austrittstemperatur von einer Synthesestufe zu der Eintrittstemperatur in die nächste Synthesestufe.

Die teilweise Kondensation von Rohmethanol aus dem Produktgas mit der gleichzeitigen Einstellung der Eintrittstemperatur in die nächste Synthesestufe wird erfindungsgemäß durch Trennung des Produktgases in zwei Teile gelöst. Ein Teil wird gekühlt um die Methanol- und Wasserdämpfe zu kondensieren, das Rohmethanol vom Synthesegas zu trennen, das kalte Synthesegas mit dem warmen Teil des Produktgases wieder zusammenzuführen, um die Einlasstemperatur in die nächste Synthesestufe zu erreichen. Die Kühlung des Produktgases bis zur Kondensation des Rohmethanols erfolgt mit Wärmerückgewinnung. Bei der Methanolsynthese kann die Wärme dem Kesselspeisewasser und/oder dem voll entsalzten Wasser übertragen werden.

Weitere Ausgestaltungen der Erfindung betreffen die Ammoniaksynthese im Speziellen. Bei der Ammoniak Synthese, wo die Temperatur des Produktgases mit mehr als 100° C größer als bei der Methanolsynthese ist, kann ein Teil der zur Kondensation abzuführenden Wärme auch zur Dampferzeugung benützt werden. Der größere Temperaturunterschied zwischen Kondensationstemperatur und Reaktoreintrittstemperatur hat Einfluss auf das Mischungsverhältnis der Teilströme. Aufgrund des sehr großen Temperaturunterschiedes muss nur ein kleiner Teilstrom abgezweigt, gekühlt und von Ammoniak befreit werden, um nach seiner Wiederzumischung zum Hauptstrom eine passende Reaktoreintrittstemperatur zu erreichen. Hierdurch wird zwar bereits eine merkliche Verbesserung gegenüber dem Stand der Technik erreicht, das Verbesserungspotential wird aber noch nicht ganz ausgeschöpft.

Entsprechend den Unteransprüchen wird daher die Temperaturdifferenz zwischen Kondensationstemperatur und Reaktoreintrittstemperatur dadurch künstlich verringert, dass eine Wiederaufheizung des Gases nach der Auskondensation des Ammoniaks erfolgt. Die Verringerung der Temperaturdifferenz bewirkt eine Änderung des Mischungsverhältnisses dahingehend, dass ein größerer Teilstrom zur Auskondensation von Ammoniak geführt werden kann, was die Menge an zusätzlich gewonnenem Ammoniak erhöht. Im Grenzfall kann es sinnvoll sein, den Teilstrom, aus dem kein Ammoniak auskondensiert wird, möglichst klein zu halten und nur zu Regelungszwecken zu betreiben.

Dadurch sinkt die Eingangskonzentration von Ammoniak im nachfolgenden Reaktor, dessen Reaktionsumsatz und Reaktoraustrittstemperatur entsprechend steigen. Durch nochmalige Anwendung der Erfindung - Teilung der Ausgangsströme mit nachfolgender Teilkondensation, Wiedererwärmung und Zusammenführung der Teilströme lässt sich aber auch hier wieder ein großer Teil des Ammoniaks gewinnen und somit durch Senkung der Ammoniakkonzentration ein Gaszustand erzeugen, der es gestattet, einen weiteren Reaktor nachzuschalten.

Da mit jedem Reaktordurchlauf bei der Ammoniakerzeugung jeweils immer nur ein kleiner Teil des Synthesegases umgesetzt werden kann, in der Regel 10 bis 20 % der Gesamtgasmenge, ist auch für weitere, nachfolgende Reaktoren stets genügend Synthesegas vorhanden. In einer weiteren Ausgestaltung der Erfindung wird daher vorgesehen, mindestens 3 Ammoniakreaktoren in Serie anzuordnen, wirtschaftlich sinnvoll ist eine Serienschaltung von 3, 4, 5, 6 oder 7 Reaktoren herkömmlicher Bauart, im Sinne dieser Erfindung wäre aber auch eine größere Anzahl von Reaktoren, wenn aus Wirtschaftlichkeitsgründen in jedem der Reaktoren nur ein jeweils geringerer Umsatz angestrebt wird, etwa bei Verwendung günstiger Katalysatoren und geringen, in der Fertigung günstigen Reaktorabmessungen.

Diese Möglichkeit der Nachrüstung vieler weiterer Reaktoren, z.B. in ein Kreislaufsystem, aber auch in ein einem Kreislaufsystem vorangeschaltetes Frischgassystem, wie es in der EP 1 339 641 B1 beschrieben wird, ist ein Vorteil der Erfindung.

In einer weiteren Ausgestaltung der Erfindung wird hinter jedem Ammoniakreaktor ein Abhitzekessel angeordnet, der einen Teil der Reaktionswärme in Nutzdampf umsetzt und auf diese Weise den Kühlmittelbedarf bei der Kondensation des Ammoniaks herabsetzt. Der Abhitzekessel kann sowohl vor als auch nach der Abzweigung in die erfindungsgemäßen Teilströme erfolgen, im letzteren Fall ist eine Anordnung ein jedem der Teilströme möglich.

In einer weiteren Ausgestaltung der Erfindung wird die Aufheizung des Teilstroms, aus dem Ammoniak auskondensiert wurde, durch eine Wärmeauskopplung aus einem nachfolgenden Reaktor unterstützt. Aus konstruktiven Gründen kann es in Einzelfällen, z.B. bei Nachrüstungen unter Verwendung bestehender Anlagenteile, auch vorteilhaft sein, diese Aufheizung nach der Zusammenführung der Teilströme zuzuführen, was gleichwertig ist.

Im folgenden werden einige Ausgestaltungsmöglichkeiten des Verfahrens mit Hilfe von Zeichnungen erläutert. Für die Methanolherstellung zeigen:
- Fig. 1: die Temperaturregelung zwischen den Synthesestufen mit Produktabscheidung und Wärmerückgewinnung.
- Fig. 2: ein Fließschema des Verfahrens mit drei adiabaten Synthesestufen.
- Fig. 3: ein Fließschema des Verfahrens mit zwei Synthesestufen.
- Fig. 3-A: eine andere Variante des Verfahrens mit zwei Synthesestufen und
- Fig. 3-B: eine weitere Variante des Verfahrens mit zwei Synthesestufen Für die Ammoniakherstellung zeigen:
- Fig. 4: eine Verschaltung des Verfahrens ohne Teilstrom-Wiederaufheizung
- Fig. 5: eine Verschaltung des Verfahrens mit Teilstrom-Wiederaufheizung und Dampferzeugung
- Fig. 6: eine Verschaltung des Verfahrens mit 5 Reaktoren

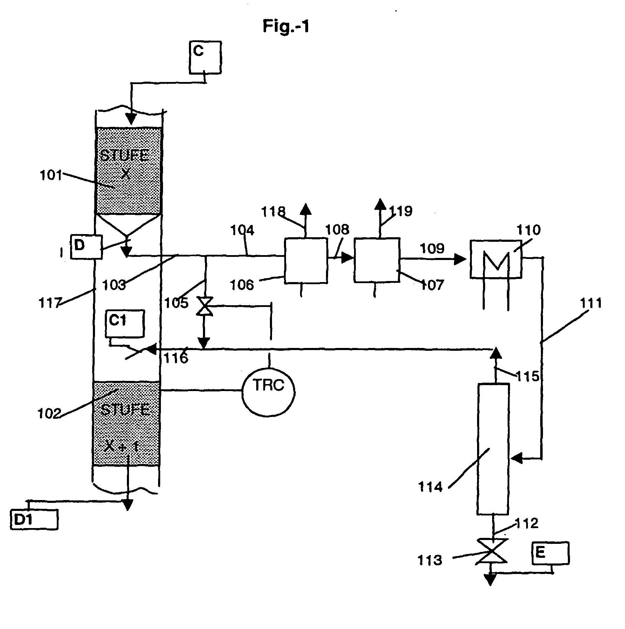

Gemäß Fig.1 wird ein Produktgemisch Strom D, in der Leitung 103 aus der Synthesestufe X 1 in zwei Teile getrennt, der erste Teil in Leitung 104 wird zum indirekten Wärmeaustauscher 106 geleitet, in dem ein Teil der enthaltenden Wärme ans Kesselspeisewasser 118 übertragen wird und durch die Leitung 108 wird es zum Wärmeaustauscher 107 geleitet, wo es die Wärme an das voll entsalzte Wasser 119 überträgt und bis 60° C abkühlt. Das vorgewärmte voll entsalzte Wasser 119 und Kesselspeisewasser 118 werden zu einer Dampferzeugungsanlage außerhalb des Synthesekreislaufs geleitet. Das erste Produktgemisch wird weiter durch die Leitung 109 dem Wasserkühler 110 zugeführt und bis 40° C abgekühlt, so dass die enthaltenen Wasser- und Methanoldämpfe kondensieren und durch die Leitung 111 in den Abscheider 114 gelangen. Im Abscheider 114 wird das flüssige Rohmethanol von den gasförmigen Bestandteilen des Produktgases 104 getrennt und durch die Leitung 112 und das Entspannungsventil 113 zu einem Sammelentspannungsbehälter weiter geleitet. Die gasförmigen Bestandteile durchlaufen den Abscheider 114 durch die Leitung 115 und werden mit dem zweiten Produktgasteil aus der Leitung 105 zusammengeführt und durch die Leitung 116 als Eintrittsstrom C1 der nächsten Synthesestufe X+1 102 zugeführt. Der Eintrittstrom C1 mit einer kleineren Temperatur als Strom D und befreit von einem Teil des Wasser und Methanolgehalts, ist somit hinsichtlich dem Gleichgewicht und der Kinetik günstiger eingestellt. Die Vorteile des Verfahrens äußern sich durch ein viel kleineres Kreislaufverhältnis als bei den bekannten adiabaten Verfahren.

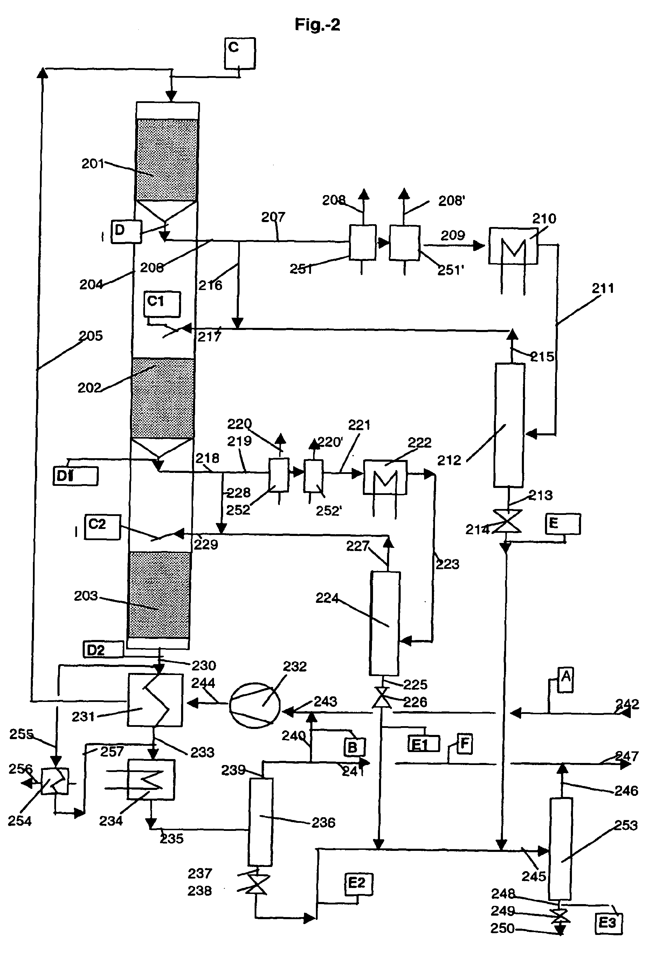

Fig. 2 zeigt eine Ausgestaltungsmöglichkeit des Verfahrens mit drei Synthesestufen 201, 202 und 203 in Serie geschaltet in einem druckbeständigen Synthesereaktorgehäuse 204. Das Frischgas, Strom A, in der Leitung 242 mischt sich mit dem Umlaufgas, Strom B, aus der Leitung 240 und wird vom Umlaufverdichter 232 angesaugt, verdichtet und durch die Leitung 244 zum Gas-Gas Wärmeaustauscher 231 gefördert. Durch Wärmeaustausch mit dem Endproduktgas aus der Synthesestufe 203, Strom D2, aus der Leitung 230 wird es bis zur Eintrittstemperatur in der ersten Synthesestufe vorgewärmt und erreicht durch die Leitung 205, als Strom C, die erste Synthesekatalysatorschicht 201, wo die Reaktion adiabatisch abläuft und die Temperatur bis zum Austritt aus dem Katalysator ständig wächst.

Das Produktgemisch Strom (D), aus der Leitung 206 wird erfindungsgemäß in zwei Teile getrennt. Der erste Teil fließt durch die Leitung 207 und den Wärmeaustauscher 251, wo es einen Teil der Wärme an das Kesselspeisewasser 208 abgibt. Anschließend zum Wärmeaustauscher 251', wo es die Wärme an das voll entsalzte Wasser 208' abgibt und bis 60° C abkühlt. Durch die Leitung 209 erreicht es den Wasserkühler 210, kühlt bis 40° C ab, die Wasser- und Methanoldämpfe kondensieren und werden von den gasförmigen Bestandteile im Abscheider 212 getrennt. Das flüssige Methanol enthaltende Gemisch wird durch die Leitung 213 und das Entspannungsventil 214 als erster Rohmethanol Produktstrom E, dem Synthesekreislauf entnommen, und zum Zwischenentspannungsbehälter 253 durch die Sammelleitung 245 geleitet. Die gasförmigen Bestandteile werden durch die Leitung 215 vom Abscheider 212 entnommen und mit dem zweiten Produktgasteil aus der Leitung 216, in die Leitung 217, zu dem Eintrittstrom C1, der Synthesestufe 202, wieder vereinigt.

Das Produktgas der zweiten Synthesestufe, Strom D1, aus der Leitung 218 wird ebenfalls in zwei Ströme geteilt, Leitung 219 und Leitung 228. Das Produktgemisch aus der Leitung 219 wird dem Wärmetauscher 252 zugeleitet, wodurch ein Teil der Wärme an das Kesselspeisewasser 220 übertragen wird, und anschließend im Wärmeaustauscher 252' wird Wärme an das voll entsalzte Wasser abgegeben und das Produktgemisch bis 60° C abgekühlt. Durch die Leitung 221 erreicht es den Wasserkühler 222, kühlt bis 40°C ab, die Wasser- und Methanoldämpfe kondensieren und werden von den gasförmigen Bestandteilen im Abscheider 224 getrennt. Das flüssige, Methanol enthaltende Kondensat wird durch die Leitung 225 und das Entspannungsventil 226 als zweites Produktgemisch E1 dem Synthesekreislauf entnommen und durch die Sammelleitung 245 dem Zwischenentspannungsbehälter 253 zugeleitet. Die gasförmigen Bestandteile aus dem Abscheider 224 werden durch die Leitung 227 entnommen und mit dem warmen Produktgemischteil aus der Leitung 228 zusammengeführt und der Synthesestufe 203 durch die Leitung 229 als Strom C2 zugeführt.

Das Mengenverhältnis der Ströme aus der Leitung 228 und der Leitung 227 ist durch die Temperaturreglung im Eintritt zur Synthesestufe 203 gesteuert. Durch die Leitung 230 wird das Produktgemisch Strom D2 aus der Synthesestufe 3 entnommen und dem Gas-Gas-Wärmeaustauscher 231 zugeführt. Hier kühlt es ab und erwärmt das Gemisch von Frischgas und Umlaufgas, welches durch die Leitung 244 der ersten Synthesestufe zugeführt wird. Ein Teil des Produktgases D2 wird durch die Leitung 255 auch zur Wärmerückgewinnung im Wärmeaustauscher 254 benutzt . Die Wärme wird an das voll entsalzte Wasser, Leitung 256 übertragen und das abgekühlte Produktgas wird durch die Leitung 257 dem Produktgas beim Austritt des Wärmeaustauschers 231 zugeleitet. Durch die Leitung 233 wird das Gemisch zum Wasserkühler 234 geleitet und bis auf 40°C gekühlt, Wasser- und Methanoldämpfe kondensieren und werden durch die Leitung 235 zum Abscheider 236 geleitet.

Hier trennt sich das flüssige, Methanol enthaltende Kondensat von den gasförmigen Bestandteilen. Das Kondensat, Strom E2, wird durch die Leitung 237 und das Entspannungsventil 238 in die Sammelleitung 245 und den Zwischenentspannungsbehälter 253 geleitet. Die gasförmigen Bestandteile werden durch die Leitung 239 aus dem Abscheider 236 entnommen und in zwei Teile getrennt. Das Umlaufgas , das durch die Leitung 240 vom Umlaufverdichter 232 angesaugt wird, und das Purgegas, das durch die Leitung 241 aus dem Synthesekreislauf entspannt wird, um die Ansammlung von Inertgasen zu verhindern. Aus dem Entspannungsbehälter 253, durch Leitung 248, dem Entspannungsventil 249 und der Leitung 250 wird das Rohmethanol zur nicht dargestellten, aber bekannten Destillation geleitet. Das vorgewärmte Kesselspeisewasser (208 und 220) sowie das vorgewärmte voll entsalzte Wasser (208', 220' und 256) werden zu einen Dampferzeuger außerhalb des Synthesekreislaufes geleitet.

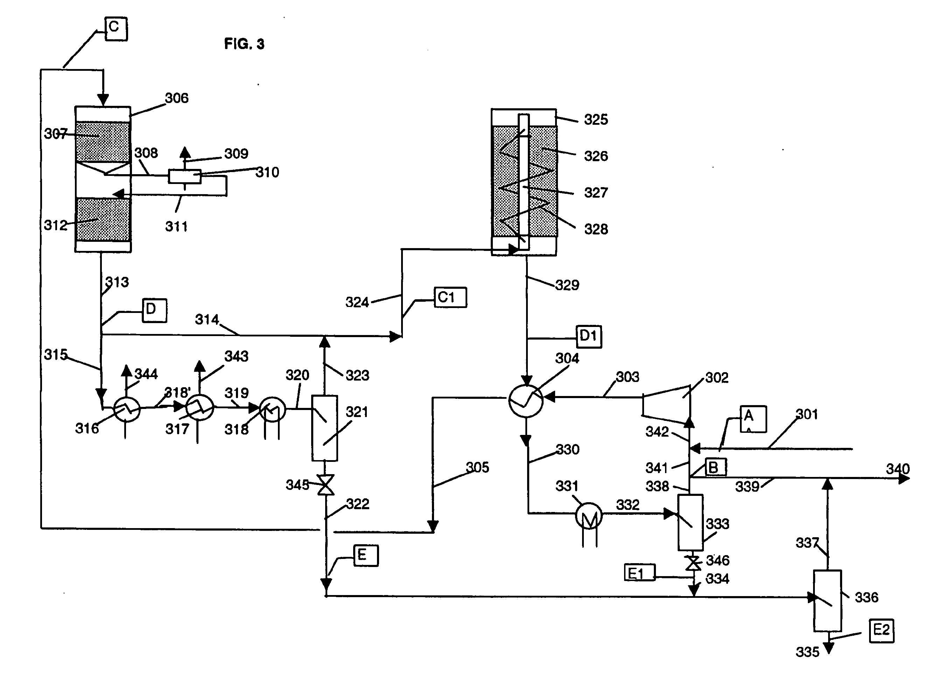

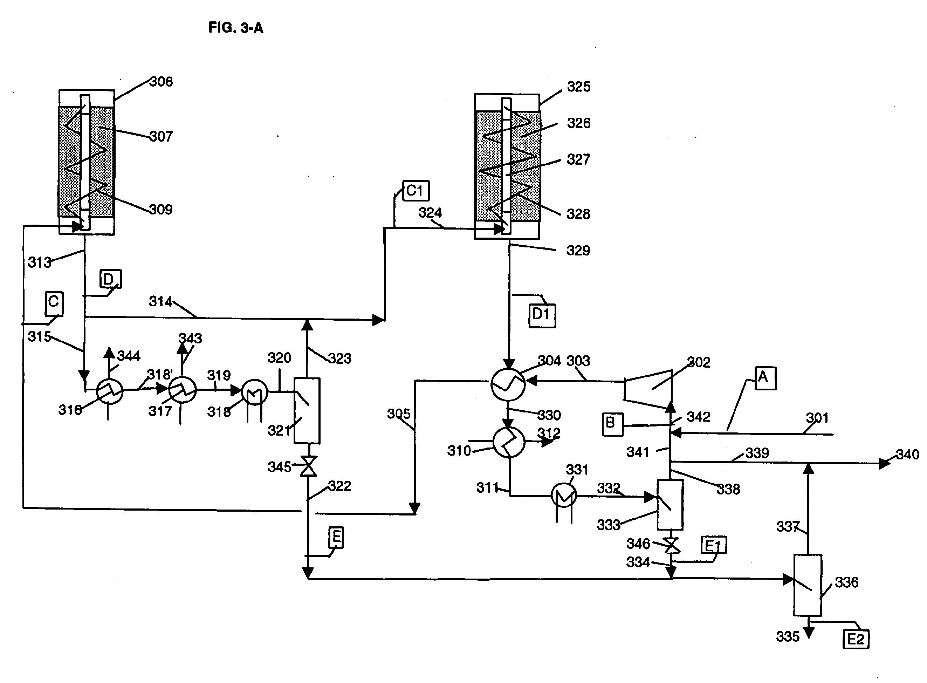

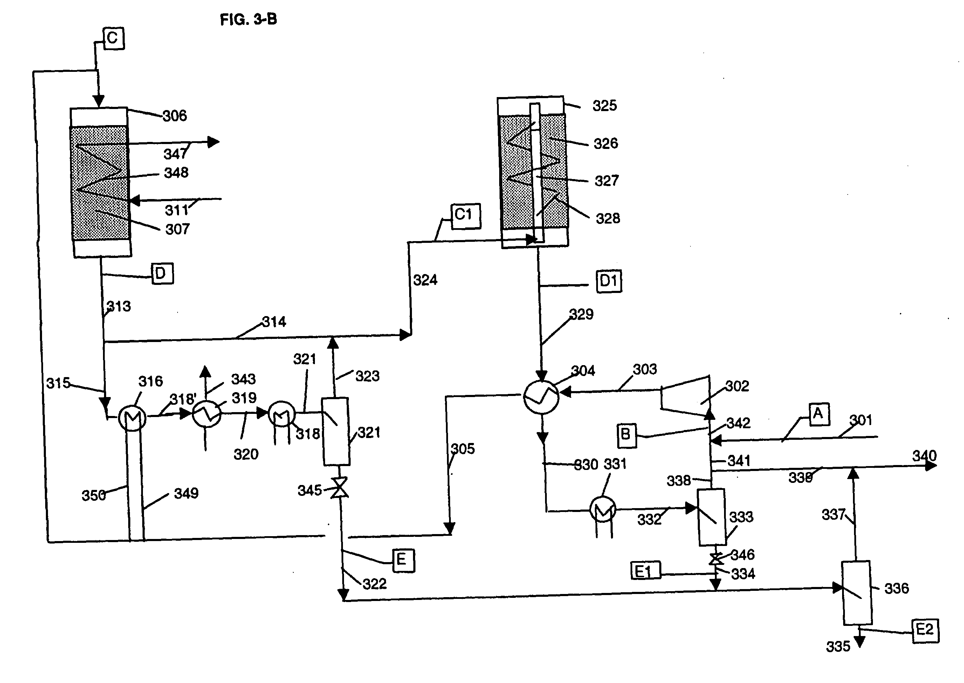

Gemäß Fig. 3 wird ein Gemisch aus frischem und zurückgeführtem Synthesegas durch die Leitung 305 als Strom C in den ersten Synthesereaktor 306 geleitet. Dieser erste Synthesereaktor ist ein bekannter, adiabatisch betriebener Schachtreaktor, in welchem der Kupferkatalysator in zwei Betten 307 und 312, mit indirekter Zwischenkühlung, angeordnet ist. Als Kühlmittel dient das Kesselspeisewasser 309, das durch den Wärmeaustauscher 310, einem Dampfgenerator in der Gaserzeugungsanlage zugeführt wird. Das Produktgemisch aus dem Katalysatorbett 307 wird durch die Leitung 308 entnommen und durch den Wärmeaustauscher 310 geleitet, übergibt einen Teil seiner Wärme an das Kesselspeisewasser, und kühlt ab bis zur Eintrittstemperatur im Katalysatorbett 312.

Durch die Leitung 313 wird der Strom D, das Produktgemisch aus dem ersten Synthesereaktor 306, entnommen und in zwei Teile getrennt. Durch die Leitung 314 wird ein Teil des Produktgemisches D warm weiter geleitet. Durch die Leitung 315 wird der andere Teil des Produktgemisches D durch die Wärmeaustauscher 316 und 317 geführt, und zuletzt durch den Wasserkühler 318 bis 40°C abgekühlt und durch Leitung 320 in den Abscheidebehälter 321 geleitet. In dem Wärmeaustauscher 316 wird mit dem Kesselspeisewasser 344 und im Wärmeaustauscher 317 mit dem voll entsalzten Wasser 343 der größte Teil der Wärme aus dem Produktgemisch rückgewonnen.

Im Abscheidebehälter 321 wird das kondensierte Methanol-Wassergemisch von den gasförmigen Bestandteilen getrennt, und durch die Leitung 322 und das Entspannungsventil 345 der erste Rohmethanol Strom E aus dem Abscheidebehälter 321 entnommen. Die gasförmigen Bestandteile strömen durch die Leitung 323 aus dem Abscheidebehälter 321 und mischen sich mit dem warmen Teil des Produktgases D aus der Leitung 314 und bilden in der Leitung 324 den Strom C1, der die Eintrittstemperatur in den Kühlröhren 328 des zweiten Synthesereaktors erreicht. Die Kühlröhren durchqueren spiralförmig den Synthesekatalysator 326 des zweiten Synthesereaktors von unten nach oben. Das Synthesegas übernimmt einen Teil der Reaktionswärme und wird so bis zur Eintrittstemperatur im Katalysator vorgewärmt und strömt anschließend von oben nach unten durch den Synthesekatalysator.

Das Produktgemisch D1 wird durch die Leitung 329 aus dem Synthesereaktor entnommen und durch den Wärmeaustauscher 304 geleitet, in dem es abkühlt und gleichzeitig das Gemisch aus frischem und zurückgeführtem Synthesegas, das vom Umlaufverdichter 302 gefördert ist, bis zur Eintrittstemperatur in den ersten Synthesereaktor als Strom C vorwärmt. Das Produktgemisch D1 strömt weiter durch die Leitung 330 in den Wasserkühler 331 wo es bis 40°C gekühlt wird und durch die Leitung 332 gelangt es in den Abscheidebehälter 333, in welchem sich die Gase von der Flüssigkeit trennen. Die gasförmigen Bestandteile werden durch die Leitung 338 entnommen, wobei man einen Teil durch die Leitung 339 aus den Verfahren entfernt. Die zurückzuführenden Synthesegase aus der Leitung 341 zusammen mit dem Frischgas aus der Leitung 301 werden vom Umlaufverdichter 302 angesaugt und in bereits beschriebener Weise zu dem ersten Synthesereaktor 306 als Strom C geleitet.

Methanolreiches Kondensat zieht man aus dem Abscheidebehälter 333 als Strom E1 durch das Entspannungsventil 346 und die Leitung 334 ab, und zusammen mit dem Strom E aus dem Abscheidebehälter 321 führt man das gesamte Rohmethanol in den Entspannungsbehälter 336. Durch die Leitung 337 zieht man ein Restgas ab und durch die Leitung 335 das Rohmethanol, das nun in bekannter Weise in einer nicht dargestellten Anlage destillativ gereinigt wird.

Fig. 3-A zeigt ein Fließschema des Verfahrens, in der beide Synthesereaktoren 306 und 325 durch indirekten Wärmeaustausch mit Synthesegas, das durch spiralförmige Röhren, die den Katalysator durchqueren fließt, gekühlt sind. Zusätzlich zu den Ausgestaltungsmöglichkeiten gemäß Fig. 3 wird noch ein Wärmeaustauscher 310 für weitere Wärmerückgewinnung aus dem zweiten Produktgas, Strom D1, vorgesehen. Durch die Leitung 330 wird das Produktgemisch D1 vom Wärmeaustauscher 304 zum Wärmeaustauscher 310 geleitet, und durch die Leitung 311 weiter zum Wasserendkühler 331. Im Wärmeaustauscher 310 wird ein Teil der Wärme vom voll entsaizten Wasser 312 übernommen und dient zur Dampferzeugung in der Synthesegaserzeugung, die hier nicht dargestellt ist. Die übrigen Bezugsziffern und Stromnummern haben die bereits beschriebene Bedeutung.

Fig. 3-B zeigt eine Ausgestaltungsmöglichkeit des Verfahrens, in dem der erste Synthesereaktor 306 ein isothermer Synthesereaktor ist. Die Katalysatorschüttung 307 ist von gewickelten Kühlröhren 348 durchzogen, in welchen als Kühlmittel unter erhöhten Druck siedendes Wasser im Natur- oder Zwangsumlauf 311 strömt. Ein Gemisch von Dampf und siedendem Wasser wird durch die Leitung 346 abgezogen und zu einer nicht dargestellten Dampftrommel geleitet. Für die Vorwärmung des Gemisches von Frischgas und Umlaufgas, Strom C, Leitung 305, bis zur Eintrittstemperatur im ersten Synthesereaktor 306, wird das Gasgemisch auch durch Wärmeaustauscher 316, mit Hilfe der Leitungen 349 und 350, geleitet. Die übrigen Bezugsziffern und Strom Nummern, haben dieselbe Bedeutung wie in Fig. 3 und Fig. 3-a.

Im folgenden wird die erfindungsgemäße Arbeitsweise der Methanolanlage an Beispielen näher erläutert, den Beispielen 2, 3 und 4 aus Tabelle 1 liegen die Anordnungen der Fig. 2, Fig. 3 beziehungsweise der Fig. 3-A zugrunde. Beispiel 1 ist demgegenüber ein Vergleichsbeispiel gemäß dem Stand der Technik und entspricht Fig. 2, jedoch ohne Rohmethanol-Abscheidung nach den Synthesestufen 201 und 202, bzw. ohne die Wärmeaustauscher 251' und 252', ohne die Wasserkühler 210 und 222, und ohne die Abscheider 212 und 224. Die Produktgase 206 und 218 werden nicht mehr in zwei Teile getrennt, sondern nach Wärmeabgabe in den Wärmeaustauschern 251 bzw. 252 direkt zur nächste Synthesestufe geleitet. Die Daten der Beispiele sind rechnerisch ermittelt worden. Zur besseren Übersicht und zum Vergleich sind die Daten tabellarisch wiedergeben.

**Tabelle 1**

| | Beispiel 1 | Beispiel 2 | Beispiel 3 | Beispiel 4 |
|---|---|---|---|---|
| **Strom A)** | | | | |
| Nm³/h | 600000 | 750000 | 600000 | 600000 |
| H₂ (Mol-%) | 71,5 | 71,5 | 71,50 | 71,50 |
| CO (Mol-%) | 16,5 | 16,5 | 16,50 | 16,50 |
| CO₂ (Mol-%) | 8,2 | 8,2 | 8,20 | 8,20 |
| Inerte (Mol-%) | 3,8 | 3,8 | 3,80 | 3,80 |
| Kreislaufverhältnis | 1:6 | 1:4,6 | 1 : 3,5 | 1 : 2,7 |

| **Strom (B)** | | | | |
|---|---|---|---|---|
| H₂ (Mol-%) | 78,38 | 78,17 | 78,35 | 78,26 |
| CO (Mol-%) | 0,60 | 1,00 | 0,56 | 0,56 |
| CO₂ (Mol-%) | 0,61 | 1,00 | 0,85 | 1,46 |

| **Strom (C)** | | | | |
|---|---|---|---|---|
| H₂ (Mol-%) | 77,40 | 76,98 | 76,83 | 76,44 |
| CO (Mol%) | 2,87 | 3,77 | 4,10 | 4,86 |
| CO₂ (MOL-%) | 1,69 | 2,29 | 2,53 | 3,28 |
| Sonstige (Mol-%) | 18,04 | 16,96 | 16,54 | 15,42 |
| Temperatur (° C) | 230,5 | 227,5 | 230 | 114,25 |
| Druck (bar) | 101 | 101 | 101 | 101 |
| Belastung (Nm³/m³.h) | 34000 | 34000 | 17420 | 14300 |

| **Strom (D)** | | | | |
|---|---|---|---|---|
| CH₃0H (Mol-%) | 2,09 | 2,16 | 4,33 | 5,43 |
| H₂ (Mol-%) | 75,87 | 75,36 | 73,62 | 72,51 |
| CO (Mol-%) | 1,86 | 2,75 | 1,68 | 1,67 |
| CO₂ (Mol-%) | 1,05 | 1,64 | 1,42 | 2,14 |
| Sonstige (Mol-%) | 19,13 | 18,09 | 18,95 | 18,25 |
| Temperatur (°C) | 276,3 | 275,3 | 283,8 | 240,3 |

| **Strom (C1)** | | | | |
|---|---|---|---|---|
| CH₃OH (Mol-%) | 2,09 | 1,83 | 2,91 | 3,27 |
| H₂ (Mol-%) | 75,87 | 75,73 | 75,1 | 74,68 |
| CO (Mol-%) | 1,86 | 2,76 | 1,71 | 1,72 |
| CO₂ (Mol-%) | 1,05 | 1,65 | 1,45 | 2,20 |
| Sonstige (Mol-%) | 19,30 | 19,03 | 18,83 | 18,13 |
| Temperatur (°C) | 240,5 | 235 | 202 | 159,7 |

| **Strom (E)** | | | | |
|---|---|---|---|---|
| CH₃OH (To/24h) | | 473,96 | 1261,6 | 1550,9 |

| **Strom (D1)** | | | | |
|---|---|---|---|---|
| CH₃OH (Mol-%) | 3,40 | 3,49 | 4,92 | 5,65 |
| H₂ (Mol-%) | 74,95 | 74,55 | 73,5 | 72,6 |
| CO (Mol-%) | 0,95 | 1,65 | 0,53 | 0,52 |
| CO₂ (Mol-%) | 0,77 | 1,3 | 0,85 | 1,35 |
| Sonstige (Mol-%) | 19,92 | 19,01 | 20,2 | 19,88 |
| Temperatur (°C) | 274,3 | 277,3 | 216 | 216 |

| **Strom (E1)** | | | | |
|---|---|---|---|---|
| CH₃OH (To/24h) | | 640,22 | 3752 | 3440 |

| **Strom (C2)** | | | | |
|---|---|---|---|---|
| CH₃OH (Mol-%) | 3,40 | 3,03 | - | |
| H2 (Mol-%) | 74,95 | 75,02 | - | |
| CO (Mol-%) | 0,95 | 1,66 | - | |
| CO₂ (Mol-%) | 0,77 | 1,31 | - | |
| Sonstige (Mol-%) | 19,92 | 18,98 | - | |
| Temperatur (°C) | 253 | 245 | - | |

| **Strom (D2)** | | | | |
|---|---|---|---|---|
| CH₃OH (Mol-%) | 4,04 | 4,23 | - | |
| H₂ (Mol-%) | 74,46 | 74,07 | - | |
| CO (Mol-%) | 0,57 | 0,95 | - | |
| CO₂ (Mol-%) | 0,58 | 0,95 | - | |
| Sonstige (Mol-%) | 20,35 | 19,80 | - | |
| Temperatur (°C) | 268,3 | 273,5 | - | |

| **Strom (E2)** | | | | |
|---|---|---|---|---|
| CH₃OH (To/24h) | 5024 | 5125,5 | 5013,6 | 4990,9 |

| **Strom (E3)** | | | | |
|---|---|---|---|---|
| CH₃OH (To/24h) | 5024 | 6240 | - | |

Die gesamte Katalysatormenge in Beispiel 1 und Beispiel 2 ist gleich. Im Beispiel 3 wird die gesamte Katalysatormenge im Verhältnis 1:1,2 zwischen den Synthesereaktoren 6 und 25 verteilt. Im Beispiel 4 wird die gesamte Katalysatormenge im Verhältnis 1:1,25 zwischen den zwei Synthesereaktoren verteilt. Im Beispiel 3 und 4 ist die Katalysatormenge mit 8% weniger als im Beispiel 1 angegeben.

Weitere Beispiele enthält Tabelle 2. In Tabelle 2 entspricht das Beispiel 5 dem Stand der Technik und Beispiel 6 entspricht der erfindungsgemäßen Arbeitsweise nach Fig.3-B. Zum Vergleich ist eine Frischgas-Zusammensetzung mit CO:CO₂ mit ca. 1:1 benutzt worden. In Beispiel 5 wird das Produktgemisch aus dem ersten Synthesereaktor 6, Strom D, aus der Leitung 313, Fig-3B nicht in zwei Teile getrennt, nicht abgekühlt, sondern direkt als Strom C1 an den oberen Teil des zweiten Synthesereaktors 325 geleitet und fließt von oben herunter durch das Katalysatorbett 326. Das Gemisch von Umlauf- und Frischgas, Strom C aus der Leitung 305, Fig-3B, wird direkt am unteren Eingang der spiralförmigen Röhren 328, Fig-3B, geleitet, fließt von unten nach oben durch die Röhren und verlässt den zweiten Synthesereaktor auf der oberen Seite und wird zum Eintritt im Katalysatorbett 307 des ersten Synthesereaktors 306 weitergeleitet. Die gesamte Rohmethanolmenge wird aus dem zweiten Produktgemisch, Strom D1, Fig-3B, nach Abkühlung aus dem Abscheider 333 entnommen.

**Tabelle 2**

| | **Beispiel 5** | **Beispiel 6** |
|---|---|---|
| **Strom (A)** | | |
| Nm³/h | 600000 | 600000 |
| H₂ (Mol-%) | 71,138 | 71,138 |
| CO (Mol-%) | 12,819 | 12,819 |
| CO₂ (Mol-%) | 12,621 | 12,621 |
| Inerte (Mol-%) | 3,422 | 3,422 |
| Kreislaufverhältnis | 1 : 2,7 | 1 :2,7 |

| **Strom (B)** | | |
|---|---|---|
| H₂ (Mol-%) | 71,82 | 71,32 |
| CO (Mol-%) | 1,99 | 1,53 |
| CO₂ (Mol-%) | 8,38 | 4,86 |

| **Strom (C)** | | |
|---|---|---|
| H₂ (Mol-%) | 71,64 | 71,27 |
| CO (Mol-%) | 4,92 | 4,58 |
| CO₂ (Mol-%) | 9,52 | 6,96 |
| Sonstige (Mol-%) | 13,92 | 17,19 |
| Temperatur (°C) | 230 | 230 |
| Druck (bar) | 82,6 | 82,6 |
| Belastung (Nm³/m³*h) | 18500 | 17100 |

| **Strom (D)** | | |
|---|---|---|
| CH₃OH (Mol-%) | 5,45 | 4,93 |
| H₂ (Mol-%) | 65,72 | 66,26 |
| CO (Mol-%) | 3,19 | 2,64 |
| CO₂ (Mol-%) | 7,55 | 5,30 |
| Sonstige (Mol-%) | 18,11 | 20,87 |
| Temperatur (°C) | 266,7 | 264 |

| **Strom (C1)** | | |
|---|---|---|
| CH₃OH (Mol-%) | 5,45 | 2,76 |
| H₂ (Mol-%) | 65,72 | 68,62 |
| CO (Mol-%) | 3,19 | 2,74 |
| CO₂ (Mol-%) | 7,55 | 5,49 |
| Sonstige (Mol-%) | 18,11 | 20,39 |
| Temperatur (°C) | 266,7 | 163,55 |

| **Strom (E)** | | |
|---|---|---|
| CH₃OH (To/24h) | | 1576,05 |

| **Strom (D1)** | | |
|---|---|---|
| CH₃OH (Mol-%) | 7,56 | 5,72 |
| H₂ (Mol-%) | 64,04 | 65,53 |
| CO (Mol-%) | 1,78 | 1,41 |
| CO₂ (Mol-%) | 7,47 | 4,47 |
| Sonstige (Mol-%) | 19,15 | 22,87 |
| Temperatur (°C) | 218 | 226 |

| **Strom (E1)** | | |
|---|---|---|
| CH₃OH (Tone/24h) | 4841 | 3461,4 |

| **Strom (E2)** | | |
|---|---|---|
| CH₃OH(To/24h) | | 5037,4 |

In den Beispielen 5 und 6 sind die Katalysatormengen gleich. In Beispiel 6 ist der CO₂ Umsatz mit 6,3 % größer als in Beispiel 5, was einen Vorteil der erfindungsgemäßen Arbeitsweise darstellt.

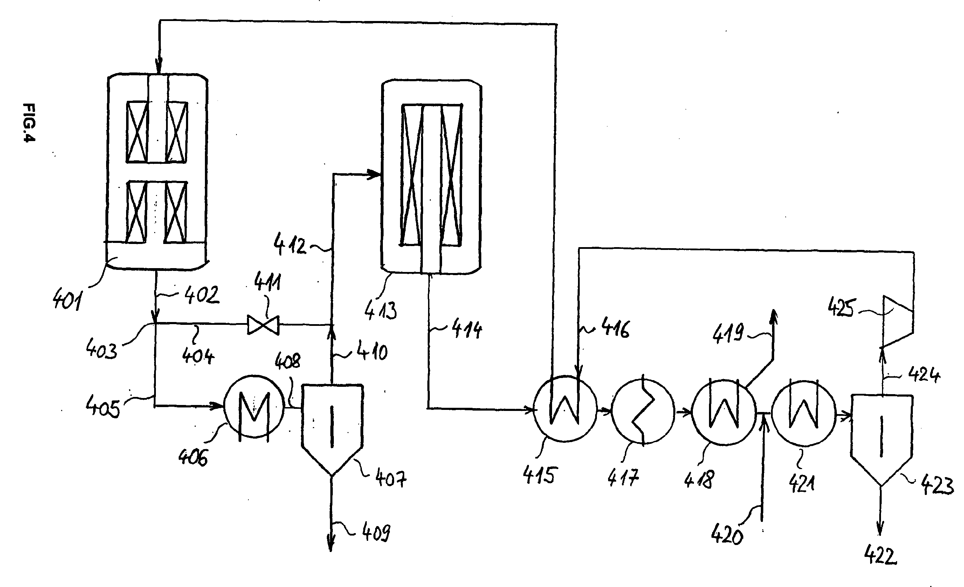

Die nachfolgenden Zeichnungen und Rechenbeispiele richten sich auf die Herstellung von Ammoniak. Fig. 4 zeigt das erfindungsgemäße Verfahren des Hauptanspruchs angewendet auf eine Ammoniakanlage herkömmlicher Bauart, in der zwei Ammoniakreaktoren innerhalb eines Synthesekreislaufes angeordnet sind. Das den ersten Synthesereaktor 401 verlassende, heiße Produktgas 402 wird an der Verzweigung 403 aufgeteilt in einen Bypassstrom 404 und einen Teilstrom 405, der in einer Kaskade von Wärmetauschern, hier symbolisch durch den Wärmetauscher 406 dargestellt, so abgekühlt wird, dass ein großer Teil des im Teilstrom 405 enthaltenden Ammoniaks auskondensiert wird, welches im Abscheider 407 aus dem abgekühlten Teilstrom 408 als flüssiges Ammoniak 409 abgezogen wird.

Der abgereicherte Teilstrom 410 wird mit dem heißen Bypassstrom 404, geregelt durch das Regelventil 411 zusammengeführt und bildet den Einsatzstrom 412 für den zweiten Synthesereaktor 413, wo die weitere Umsetzung zu Ammoniak durchgeführt wird. Das den zweiten Synthesereaktor 413 verlassende heiße Produktgas 414 wird im Gas-Gas-Wärmetauscher 415 gegen das aufzuwärmende Einsatzgas 416 des ersten Synthesereaktors 401 abgekühlt, dann üblicherweise einem Dampferzeuger 417 zugeführt, im Kühlersystem 418 auf Umgebungstemperatur abgekühlt, ein Purgegas 419 wird abgezogen, Frischgas 420 wird zugefügt, das Produktgas wird im Chiller 421 tiefgekühlt und flüssiges Ammoniak 422 wird im Ammoniakabscheider 423 abgeschieden. Das kalte Synthesegas 424 wird vom Kreislaufverdichter 425 zum Gas-Gas-Wärmetauscher 415 gefördert, wo es vor seinem Einsatz im ersten Synthesereaktor 401 wieder aufgeheizt wird.

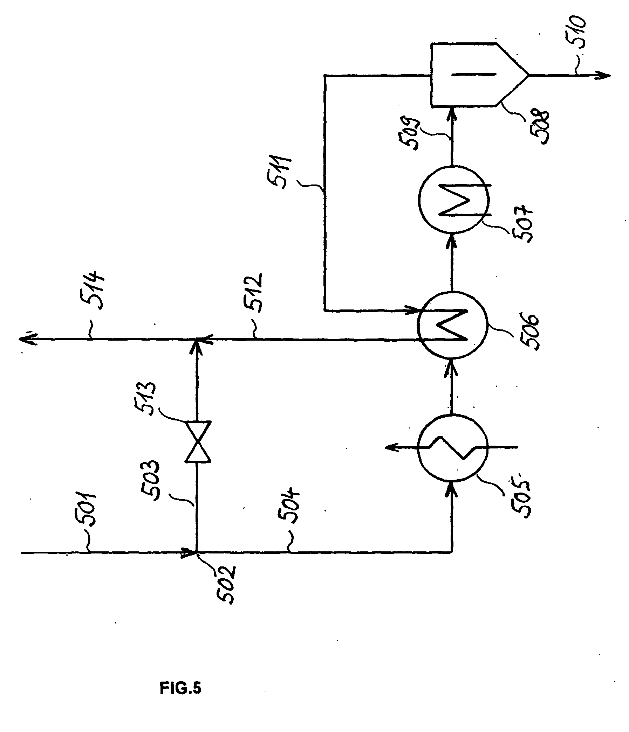

Fig. 5 zeigt das erfindungsgemäße Verfahren des Anspruchs 3, angewendet auf eine Ammoniakanlage herkömmlicher Bauart wie in Fig. 4 gezeigt, wobei die Synthesereaktoren sowie die vor der Kreislaufverdichtung gelegene Ammoniakausschleusung nicht gezeigt werden müssen, da sie mit Fig. 4 identisch sind. Das den ersten Synthesereaktor verlassende, heiße Produktgas 501 (entspricht 402) wird an der Verzweigung 502 (entspricht 403) aufgeteilt in einen Bypassstrom 503 (entspricht 404) und einen Teilstrom 504 (entspricht 405). Dieser Teilstrom 504 wird zunächst einem Dampferzeuger 505 und dann einem Gas-Gas-Wärmetauscher 506 zugeführt, der den größten Teil der Abkühlung bewirkt. Im Nachkühlsystem 507 (entspricht 406) wird der Teilstrom 504 so abgekühlt, dass ein großer Teil des enthaltenden Ammoniaks auskondensiert wird, welches im Abscheider 508 (entspricht 407) aus dem abgekühlten Teilstrom 509 (entspricht 408) als flüssiges Ammoniak 510 (entspricht 409) abgezogen wird.

Der abgereicherte Teilstrom 511 wird im Gas-Gas-Wärmetauscher 506 wieder aufgeheizt und erst anschließend als Zumischstrom 512 mit dem heißen Bypassstrom 503, geregelt durch das Regelventil 513 zusammengeführt und bildet den Einsatzstrom 514 für den nächsten Synthesereaktor (hier nicht gezeigt). Durch Wahl des Mischungsverhältnisses der Ströme 503 und 504 sowie Wahl des Kühlsystems 507 lassen sich im Strom 514 beliebig geringe Ammoniakkonzentrationen einstellen. Je geringer die Ammoniakkonzentration im Strom 514 gewählt wird, desto größer ist der im nachfolgenden Reaktor zu erwartende Temperaturanstieg, und desto geringer ist die erforderliche Reaktoreintrittstemperatur.

Der Fachmann erhält auf diese Weise einen Freiheitsgrad bei Nachrüstungsmaßnahmen, bei denen er vorhandene Reaktoren weiternutzen soll. Niedrige Eintrittstemperaturen verwendet er bevorzugt bei Reaktoren, die mit zwei Katalysatorbetten und einen inneren Wärmetausch ausgestattet sind. Höhere Eintrittstemperaturen verwendet er bei Reaktoren mit einem einzigen Katalysatorbett. Bei der Wahl niedriger Eintrittstemperatur ist das Mischungsverhältnis der Ströme 503-zu-504 möglichst klein und kann auch den Grenzfall Null erreichen, und der Anteil des Ammoniaks, welches aus dem Strom 509 abgeschieden werden soll, ist möglichst groß. In diesem Fall kann im Dampferzeuger 505 viel Hochdruckdampf erzeugt werden, da ein erheblicher Energieüberschuss vorliegt.

Bei der Wahl höherer Eintrittstemperatur ist das Mischungsverhältnis der Ströme 503-zu-504 relativ groß und der Anteil des Ammoniaks, welches aus dem Strom 509 abgeschieden werden soll, kleiner. In diesem Fall kann im Dampferzeuger 505 nur wenig Hochdruckdampf erzeugt werden, da kein erheblicher Energieüberschuss vorliegt. Allerdings sind die Aufwendungen in Reaktoren, Wärmetauscher und Ammoniakkondensation geringer. Bei der Planung von Nachrüstungsmaßnahmen mit einer Vielzahl von Reaktoren kann der Fachmann auch zwischen den einzelnen Reaktoren der Erfindung gemäße, unterschiedliche Konzepte vorsehen, wobei jeder bekannte Reaktortyp entsprechend einsetzbar ist.

Die Vorteile der Erfindung werden auch anhand der folgenden 4 Rechenbeispiele 7-10, die in Tabelle 3 zusammengefasst sind, sichtbar. Beispiel 7 ist dabei das Vergleichsbeispiel nach dem herkömmlichen Stand der Technik und entspricht der in Fig. 4 gezeigten Konfiguration, bei der der Teilstrom 405 den Grenzfall mit Nulldurchsatz darstellt. Beispiel 8 zeigt die Konfiguration von Fig. 4 unter Anwendung der Erfindung entsprechend dem Hauptanspruch. Beispiel 9 zeigt eine Anwendung nach Anspruch 3, wie sie in Fig. 5 gezeigt wird, wenn man den Strom 514 danach mit hoher Reaktoreintrittstemperatur in einen Reaktor mit einem Katalysatorbett führt. Beispiel 10 zeigt ebenfalls eine Anwendung nach Anspruch 3, wie sie in Fig. 5 gezeigt wird, wenn man den Strom 514 danach mit niedriger Reaktoreintrittstemperatur in einen Reaktor mit zwei Katalysatorbetten und innerem Wärmetausch führt.

**Tabelle 3**

| Beispiel 7 | | | | | | |
|---|---|---|---|---|---|---|
| | | **420** | **402** | **412** | **419** | **422** |
| CH₄ | Mol-% | 1,17 | 9,27 | 9,27 | 13,66 | |
| Ar | Mol-% | 0,36 | 3,11 | 3,11 | 4,20 | |
| H₂ | Mol-% | 73,54 | 50,94 | 50,94 | 55,81 | |
| N₂ | Mol-% | 24,93 | 20,49 | 20,49 | 22,32 | |
| NH₃ | Mol-% | | 16,19 | 16,19 | 4,00 | 100 |
| Summe | | 100,00 | 100,00 | 100,00 | 100,00 | |
| Durchsatz | kmol/h | 10856 | 34284 | 34284 | 930 | 4957 |
| Druck | bar | 198 | | | | |
| Temperatur | °C | 46 | 480 | 412 | 4 | 0 |

| Beispiel 8 | | | | | | |
|---|---|---|---|---|---|---|
| | | **420** | **402** | **412** | **419** | **422** |
| CH₄ | Mol-% | 1,17 | 9,27 | 9,47 | 13,66 | |
| Ar | Mol-% | 0,36 | 3,11 | 3,18 | 4,20 | |
| H₂ | Mol-% | 73,54 | 50,94 | 52,09 | 55,81 | |
| N₂ | Mol-% | 24,93 | 20,49 | 20,95 | 22,32 | |
| NH₃ | Mol-% | | 16,19 | 14,31 | 4,00 | 100 |
| Summe | | 100,00 | 100,00 | 100,00 | 100,00 | |
| Durchsatz | kmol/h | 11247 | 34284 | 33519 | 963 | 4370 |
| Druck | bar | 198 | | | | |
| Temperatur | °C | 46 | 480 | 410 | 4 | 0 |
| | | **405** | **404** | **409** | **410** | |
| CH₄ | Mol-% | 9,27 | 9,27 | | 10,58 | |
| Ar | Mol-% | 3,11 | 3,11 | | 3,55 | |
| H₂ | Mol-% | 50,94 | 50,94 | | 58,36 | |
| N₂ | Mol-% | 20,49 | 20,49 | | 23,47 | |
| NH₃ | Mol-% | 16,19 | 16,19 | 100 | 4,04 | |
| Summe | | 100,00 | 100,00 | 100,00 | 100,00 | |
| Durchsatz | kmol/h | 5969 | 28315 | 765 | 5204 | |
| Druck | bar | | | | | |
| Temperatur | °C | 480 | 480 | 0 | 0 | |

| Beispiel 9 | | | | | | |
|---|---|---|---|---|---|---|
| | | **420** | **501** | **514** | **419** | **422** |
| CH₄ | Mol-% | 1,17 | 9,27 | 9,88 | 13,66 | |
| Ar | Mol-% | 0,36 | 3,11 | 3,32 | 4,20 | |
| H₂ | Mol-% | 73,54 | 50,94 | 54,39 | 55,81 | |
| N₂ | Mol-% | 24,93 | 20,49 | 21,88 | 22,32 | |
| NH₃ | Mol-% | | 16,19 | 10,53 | 4,00 | 100 |
| Summe | | 100,00 | 100,00 | 100,00 | 100,00 | |
| Durchsatz | kmol/h | 12267 | 34284 | 32089 | 1050 | 3406 |
| Druck | bar | 198 | | | | |
| Temperatur | °C | 46 | 480 | 410 | 4 | 0 |
| | | **504** | **503** | **510** | **511** | |
| CH₄ | Mol-% | 9,27 | 9,27 | | 10,58 | |
| Ar | Mol-% | 3,11 | 3,11 | | 3,55 | |
| H₂ | Mol-% | 50,94 | 50,94 | | 58,36 | |
| N₂ | Mol-% | 20,49 | 20,49 | | 23,47 | |
| NH₃ | Mol-% | 16,19 | 16,19 | 100 | 4,04 | |
| Summe | | 100,00 | 100,00 | 100,00 | 100,00 | |
| Durchsatz | kmol/h | 17142 | 17142 | 2195 | 14947 | |
| Druck | bar | | | | | |
| Temperatur | °C | 480 | 480 | 0 | 0 | |

| Beispiel 10 | | | | | | |
|---|---|---|---|---|---|---|
| | | **420** | **501** | **514** | **419** | **422** |
| CH₄ | Mol-% | 1,17 | 9,27 | 10,58 | 13,66 | |
| Ar | Mol-% | 0,36 | 3,11 | 3,55 | 4,20 | |
| H₂ | Mol-% | 73,54 | 50,94 | 58,36 | 55,81 | |
| N₂ | Mol-% | 24,93 | 20,49 | 23,47 | 22,32 | |
| NH₃ | Mol-% | | 16,19 | 4,04 | 4,00 | 100 |
| Summe | | 100,00 | 100,00 | 100,00 | 100,00 | |
| Durchsatz | kmol/h | 15741 | 34284 | 29896 | 1348 | 2799 |
| Druck | bar | 198 | | | | |
| Temperatur | °C | 46 | 480 | 305 | 4 | 0 |
| | | **504** | **503** | **510** | **511** | |
| CH₄ | Mol-% | 9,27 | 9,27 | | 10,58 | |
| Ar | Mol-% | 3,11 | 3,11 | | 3,55 | |
| H₂ | Mol-% | 50,94 | 50,94 | | 58,36 | |
| N₂ | Mol-% | 20,49 | 20,49 | | 23,47 | |
| NH₃ | Mol-% | 16,19 | 16,19 | 100 | 4,04 | |
| Summe | | 100,00 | 100,00 | 100,00 | 100,00 | |
| Durchsatz | kmol/h | 34284 | 0 | 4388 | 29896 | |
| Druck | bar | | | | | |
| Temperatur | °C | 480 | 480 | 0 | 0 | |

Wie man aus den Beispielen in Tabelle 3 sieht, werden die folgenden Ammoniakmengen produziert:

| | | | | |
|---|---|---|---|---|
| Beispiel 7: | (Vergleichsfall) | | 4957 kmol/h = | 100 % |
| Beispiel 8: | 765 kmol/h + | 4370 kmol/h = | 5135 kmol/h = | 103,6 % |
| Beispiel 9: | 2195 kmol/h + | 3406 kmol/h = | 5601 kmol/h = | 113,0 % |
| Beispiel 10: | 2799 kmol/h + | 4388 kmol/h = | 7187 kmol/h = | 145,0 % |

Schon mit einer Schaltung aus nur 2 Reaktoren ist eine nachträgliche Kapazitätsvergrößerung von fast 50 % möglich.

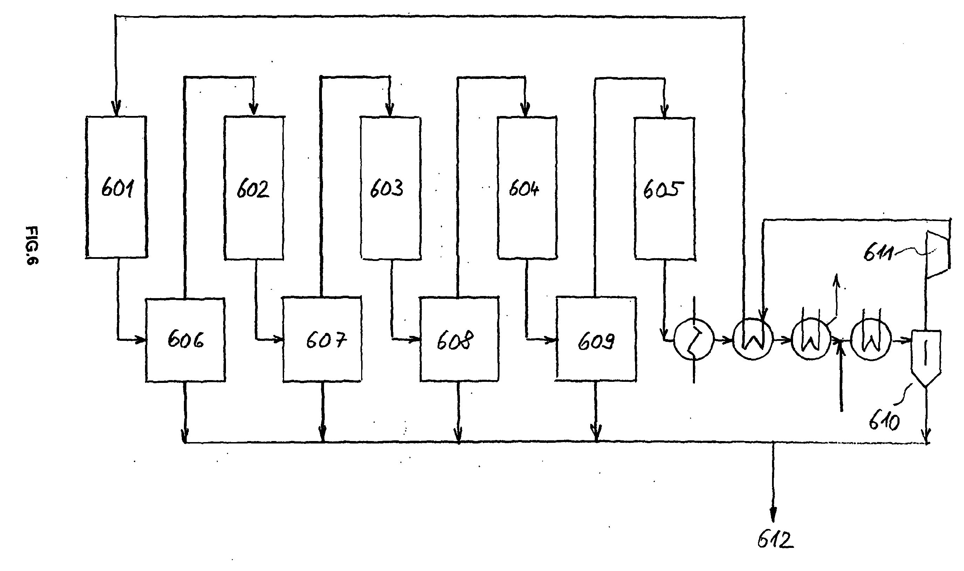

Fig. 6 zeigt im folgenden, wie man aus den Beispielen 9 und 10 folgend eine Kapazitätsvergrößerung, die den Faktor 2 übersteigt, erreichen kann. Hierbei werden die 5 Ammoniak-Reaktoren 601, 602, 603, 604 und 605 in Reihe geschaltet, wobei es nicht problematisch ist, wenn bereits 2 ursprünglich vorhandene Reaktoren weiterverwendet werden. Die Reaktoren können exakt baugleich sein oder jeder verschieden, je nach Einzelfall. Ammoniak 612 wird aus den Teilkondensationssystemen 606, 607, 608 und 609, welche alle der in Fig. 5 gezeigten Schaltung entsprechen, gewonnen, ferner nach dem letzten Reaktor aus dem Abscheider 610, wobei wegen des Kreisgasverdichters 611 hinter dem letzten Reaktor kein Bypass angeordnet sein kann.

## Patentansprüche

1. Verfahren zur Durchführung heterogen katalytischer exothermer Gasphasenreaktionen bei erhöhter Temperatur und erhöhtem Druck, wobei man das Synthesegas, bestehend aus einem Gemisch von Frischgas und/oder Umlaufgas durch mindestens zwei in einen Synthesesystem in Serie geschaltete Synthesestufen leitet,
**dadurch gekennzeichnet dass**,
• die Produktgase aus den Synthesestufen, mit Ausnahme der letzten Stufe, in mindestens zwei Teilströme getrennt werden,
• wobei ein Teilstrom soweit gekühlt wird, bis Produkt auskondensiert und das produkthaltige Kondensat von den gasförmigen Bestandteilen getrennt wird,
• und anschließend die gasförmigen Bestandteile mit dem warmen Teil des Produktgases zusammengeführt werden, um die Eintrittstemperatur der nächsten Synthesestufe zu erreichen.

2. Verfahren nach Anspruch 1, **dadurch gekennzeichnet dass**, der Teilstrom, aus dem Produkt auskondensiert und entfernt wurde, vor seiner Wiederzumischung zum warmem Teil des Produktgases erwärmt wird, wobei die Temperatur nach der Erwärmung unter der des warmem Teils des Produktgases liegt.

3. Verfahren nach Anspruch 2, **dadurch gekennzeichnet dass**, die Wärme, die zur Erwärmung des Teilstroms, der aus dem Produkt auskondensiert wurde, genutzt wird, diesem Teilstrom bei seiner Abkühlung wenigstens teilweise entzogen wird.

4. Verfahren nach einem der Ansprüche 1 bis 3, **dadurch gekennzeichnet dass**, aus dem abzukühlenden Teil des Produktgases der größte Teil der Wärme durch indirekten Wärmeaustausch mit Kesselspeisewasser und/oder voll entsalztem Wasser rückgewonnen wird.

5. Verfahren nach einem der Ansprüche 1 bis 4, **dadurch gekennzeichnet, dass** das in den ersten Synthesereaktor geleitete Synthesegas zu 16 bis 40 Mol.-% aus frischem Synthesegas besteht.

6. Verfahren nach einem der Ansprüche 1 bis 5, **dadurch gekennzeichnet, dass** der letzte Reaktor eines Synthesesystems in indirekten Wärmeaustausch mit Synthesegas gekühlt wird.

7. Verfahren nach einem der Ansprüche 1 bis 6, **dadurch gekennzeichnet, dass** aus einem Wasserstoff, Kohlenmonoxid und Kohlendioxid enthaltenden Synthesegas durch Umsetzung an körnigen, kupferhaltigen Katalysatoren bei Drücken im Bereich von 20 bis 120 bar und Temperaturen im Bereich von 200 bis 350° C Methanol erzeugt wird und dass sich zwischen der ersten und der letzten Synthesestufe 15 bis 50 % der gesamten Methanolproduktion in dem abgeschiedenen methanolhaltigen Kondensat befinden.

8. Verfahren nach Anspruch 7, **dadurch gekennzeichnet, dass** der erste Synthesereaktor ein adiabater Mehrbettreaktor ist.

9. Verfahren nach Anspruch 7, **dadurch gekennzeichnet, dass** der erste Synthesereaktor ein Röhrenreaktor mit Synthesegaskühlung ist.

10. Verfahren nach Anspruch 7, **dadurch gekennzeichnet, dass** der erste Synthesereaktor ein isothermer Reaktor ist,

11. Verfahren nach Anspruch 10, **dadurch gekennzeichnet, dass** der Katalysator von Röhren, in denen unter erhöhtem Druck siedendes Wasser als Kühlmittel fließt, durchzogen ist.

12. Verfahren nach einem der Ansprüche 2 bis 6, **dadurch gekennzeichnet, dass** mindestens 3 Ammoniakreaktoren in Serie geschaltet werden.

13. Verfahren nach einem der Ansprüche 1 bis 6 sowie 12, **dadurch gekennzeichnet, dass** hinter jedem Ammoniakreaktor ein Abhitzekessel angeordnet wird, der einen Teil der Reaktionswärme in Nutzdampf umsetzt.

14. Verfahren nach einem der Ansprüche 2 bis 6 sowie 12, **dadurch gekennzeichnet, dass** die Aufheizung des Teilstroms, aus dem Ammoniak auskondensiert wurde, durch eine Wärmeauskopplung aus einem nachfolgenden Reaktor unterstützt wird.

## Claims

1. Method for carrying out heterogeneous catalytic exothermic gas phase reactions at high temperature and pressure, wherein the synthesis gas comprised of a mixture of make-up gas and/or of recycle gas is fed through at least two synthesis stages that are connected in-series to form a synthesis system,
**characterized in that**
- the product gases from the synthesis stages, with the exception of the last stage, are separated into at least two partial flows,
- one partial flow is cooled until the product is condensated out, and the condensate containing the product is separated from the gaseous constituents,
- and, subsequently, the gaseous constituents are combined with the warm portion of the product gas in order to reach the inlet temperature of the following synthesis stage.

2. Method according to claim 1, **characterized in that** the partial flow, from which the product has been condensated out and removed, is heated before its remixing with the warm portion of the product gas, wherein the temperature after heating is lower than the temperature of the warm portion of the product gas.

3. Method according to claim 2, **characterized in that** the heat used for heating the partial flow, from which the product has been condensated out, is at least partially recovered from the cooling of this partial flow.

4. Method according to any of claims 1 to 3, **characterized in that** from the portion of the product gas to be cooled, most of the heat is recovered by indirect heat exchange with boiler feed water and/or totally desalinated water.

5. Method according to any of claims 1 to 4, **characterized in that** the synthesis gas led into the first synthesis reactor is composed of 16 to 40 mol-% of make-up synthesis gas.

6. Method according to any of claims 1 to 5, **characterized in that** the last reactor of a synthesis system is cooled by indirect heat exchange with synthesis gas.

7. Method according to any of claims 1 to 6, **characterized in that** methanol is obtained from a synthesis gas containing hydrogen, carbon monoxide and carbon dioxide, by conversion on granular, copper containing catalysts, at pressures between 20 and 120 bar and temperatures between 200 and 350°C and **in that**, between the first and last synthesis stage, 15 to 50% of the total methanol production is present in the separated methanol containing condensate.

8. Method according to claim 7, **characterized in that** the first synthesis reactor is an adiabatic multiple bed reactor.

9. Method according to claim 7, **characterized in that** the first reactor is a tube reactor with synthesis gas cooling.

10. Method according to claim 7, **characterized in that** the first synthesis reactor is an isothermal reactor.

11. Method according to claim 10, **characterized in that** the catalyst is crossed by tubes, in which water, boiling at elevated pressure, flows.

12. Method according to any of claims 2 to 6, **characterized in that** at least 3 ammonia reactors are connected in series.

13. Method according to any of claims 1 to 6 and 12, **characterized in that** downstream of each ammonia reactor a cooling vessel is arranged, which converts part of the reaction heat into working steam.

14. Method according to any of claims 2 to 6 and 12, **characterized in that** heating of the partial flow, from which ammonia has condensated out, is supported by heat transfer from a successive reactor.

## Revendications

1. Procédé de mise en oeuvre de réactions en phase gazeuse exothermiques catalytiques hétérogènes à température et sous pression élevées, dans lequel le gaz de synthèse, composé d'un mélange de gaz frais et/ou de gaz de recyclage, est envoyé au travers d'au moins deux étages de synthèse montés en série dans un système de synthèse,
**caractérisé en ce que**
- les gaz produits des étages de synthèse, exception faite du dernier étage, sont séparés en au moins deux flux partiels,
- un flux partiel étant refroidi jusqu'à ce que du produit soit condensé et le condensat renfermant le produit soit séparé des constituants gazeux,
- puis les constituants gazeux sont réunis avec la partie chaude du gaz produit, pour obtenir la température d'entrée de l'étage de synthèse suivant.

2. Procédé suivant la revendication 1, **caractérisé en ce que** le flux partiel, à partir duquel du produit a été condensé et éliminé, est réchauffé avant son nouveau mélange avec la partie chaude du gaz produit, la température après le réchauffage se situant au-dessous de celle de la partie chaude du gaz produit.

3. Procédé suivant la revendication 2, **caractérisé en ce que** la chaleur utilisée pour le réchauffage du flux partiel, à partir duquel a été condensé du produit, est soutirée au moins en partie de ce flux partiel lors de son refroidissement.

4. Procédé suivant l'une des revendications 1 à 3, **caractérisé en ce que** la majeure partie de la chaleur est récupérée de la partie à refroidir du gaz produit par échange thermique indirect avec de l'eau d'alimentation de chaudière et/ou de l'eau totalement déminéralisée.

5. Procédé suivant l'une des revendications 1 à 4, **caractérisé en ce que** le gaz de synthèse envoyé dans le premier réacteur de synthèse se compose de 16 à 40% molaire de gaz de synthèse frais.

6. Procédé suivant l'une des revendications 1 à 5, **caractérisé en ce que** le dernier réacteur d'un système de synthèse est refroidi en échange de chaleur indirect avec du gaz de synthèse.

7. Procédé suivant l'une des revendications 1 à 6, **caractérisé en ce que** du méthanol est produit à partir d'un gaz de synthèse renfermant de l'hydrogène, du monoxyde de carbone et du gaz carbonique, par conversion sur des catalyseurs granuleux renfermant du cuivre, sous des pressions dans la plage de 20 à 120 bars et à des températures dans la plage de 200 à 350°C, et que 15 à 50% de la production totale de méthanol entre le premier et le dernier étage de synthèse se situent dans le condensat séparé renfermant du méthanol.

8. Procédé suivant la revendication 7, **caractérisé en ce que** le premier réacteur de synthèse est un réacteur à lits multiples adiabatique.

9. Procédé suivant la revendication 7, **caractérisé en ce que** le premier réacteur de synthèse est un réacteur tubulaire avec refroidissement du gaz de synthèse.

10. Procédé suivant la revendication 7, **caractérisé en ce que** le premier réacteur de synthèse est un réacteur isotherme.

11. Procédé suivant la revendication 10, **caractérisé en ce que** le catalyseur est traversé par des tubes, dans lesquels s'écoule de l'eau en ébullition sous pression élevée en tant qu'agent réfrigérant.

12. Procédé suivant l'une des revendications 2 à 6, **caractérisé en ce qu'**au moins trois réacteurs d'ammoniac sont montés en série.

13. Procédé suivant l'une des revendications 1 à 6 et 12, **caractérisé en ce qu'**une chaudière de récupération, qui convertit une partie de la chaleur de réaction en vapeur utile, est disposée en aval de chaque réacteur d'ammoniac.

14. Procédé suivant l'une des revendications 2 à 6 et 12, **caractérisé en ce que** le chauffage du flux partiel, à partir duquel a été condensé de l'ammoniac, est assisté par un découplage de chaleur d'un réacteur suivant.
